# EUROPÄISCHE PATENTSCHRIFT

(11) **EP 3 671 758 B1**
(45) Veröffentlichungstag und Bekanntmachung des Hinweises auf die Patenterteilung: **10.06.2026**
(21) Anmeldenummer: 18214617.5
(22) Anmeldetag: 20.12.2018
(51) Int. Cl.: G16H 40/63

(54) **VERFAHREN ZUR KONTROLLE DER NUTZUNG EINER MEDIZINISCHEN INFRASTRUKTUR**
METHOD FOR MONITORING THE USE OF A MEDICAL INFRASTRUCTURE
PROCÉDÉ DE SURVEILLANCE DE L'UTILISATION D'UNE INFRASTRUCTURE MÉDICALE

(43) Veröffentlichungstag der Anmeldung: 24.06.2020
(73) Patentinhaber: Siemens Healthineers AG, 91301 Forchheim (DE)
(72) Erfinder: Thesen, Stefan, 91077 Dormitz (DE)
(74) Vertreter: Siemens Healthineers Patent Attorneys

(56) Entgegenhaltungen:
- US-A1- 2015 201 895
- US-B1- 7 103 776

## Beschreibung

Die Erfindung betrifft ein Verfahren zur Kontrolle der Nutzung einer medizinischen Infrastruktur, wobei eine Nutzungsberechtigung eingerichtet wird, und eine Nutzung der Infrastruktur durch die Nutzungsberechtigung ermöglicht wird.

Die Kontrolle einer Nutzung von medizinischer Infrastruktur, also einerseits von Geräten, insbesondere zur medizinischen Bildgebung oder zur medizinisch-biochemischen Analyse von Proben, andererseits von spezieller Software wie zum Beispiel Analyse- und/oder Auswerte-Programme für medizinische Daten, erfolgt oftmals in einem Spannungsfeld, in dem sowohl die berechtigten Interessen eines Herstellers der medizinischen Infrastruktur als auch die Interessen des Nutzers, also beispielsweise eines Krankenhauses, eine Rolle spielen. Um Entwicklungskosten und die bisweilen nicht unerheblichen Kosten von medizinrechtlichen Zulassungsverfahren für ein Produkt decken zu können, ist es für den Hersteller einer medizinischen Infrastruktur oftmals nützlich, die Infrastruktur in ihren Funktionen und ihrer Anwendbarkeit einerseits möglichst vielseitig und flexibel einsetzbar auszugestalten, andererseits aber von einem Nutzer auch nur die tatsächlich erfolgte Nutzung der Infrastruktur zu verrechnen bzw. nur Teilfunktionen anzubieten.

Dies kann beispielsweise technisch durch einen entsprechenden Lizensierungsmechanismus erfolgen, welcher einem Nutzer lediglich die vorab gebuchten Funktionen der Infrastruktur zur Verfügung stellt, wobei zusätzlich die Anzahl und/oder der Umfang der einzelnen Nutzungsvorgänge für eine entsprechende Abrechnung mit dem Hersteller erfasst werden können. So kann beispielsweise ein Computertomograf (CT) in einem onkologischen Zentrum auf diejenigen Abbildungsmöglichkeiten eingeschränkt sein, welche für die dort anfallenden üblichen Diagnosen relevant sind, wobei die mit dem CT prinzipiell gegebene Funktionalität eines großflächigen und/oder besonders schnellen Scans, beispielsweise des ganzen Torso eines Patienten, wie er in der Unfallchirurgie etwa durchgeführt wird, deaktiviert sein kann.

Auf der Seite eines Nutzers einer medizinischen Infrastruktur, also beispielsweise in einem Krankenhaus oder einer Arztpraxis, ist eine Einschränkung eines Zugangs zur Nutzung von medizinischer Infrastruktur vorrangig aus Sicherheitsgründen erwünscht: Die Bedienung von bildgebenden medizinischen Geräten, welche zur Bildgebung Röntgenstrahlen verwenden, oder auch die Bedienung von medizinisch-biochemischen Analysegeräten, welche eine Analyse von Proben mit teilweise für den Menschen giftigen Substanzen durchführen, darf aus Sicherheitsgründen nur von entsprechend geschultem Fachpersonal durchgeführt werden. Aus diesem Grund ist auf einer Nutzer-Ebene der medizinischen Infrastruktur der Zugang zur selbigen möglichst strikt auf derartiges Fachpersonal und gegebenenfalls auf entsprechende Fachärzte zu beschränken, und die Beschränkung streng zu kontrollieren.

In einem eintretenden Notfall kann es jedoch erwünscht sein, die genannten Beschränkungen einer Nutzung der medizinischen Infrastruktur vorübergehend außer Kraft zu setzen. Ein derartiger Notfall kann beispielsweise in einer akuten Epidemie, einem Katastrophenfall oder einem terroristischen Großangriff bestehen, in welchem vorübergehend sämtliche irgend möglichen medizinischen Kapazitäten der gesamten Gesundheitsversorgung möglichst vollständig auszuschöpfen sind. In einem solchen Fall gilt es einerseits, die durch den Hersteller im Rahmen eines Lizensierungsmechanismus implementierten Beschränkungen einer Nutzung der Infrastruktur auszusetzen, und vorteilhafterweise auch denjenigen Mitarbeitern eines Krankenhauses oder einer Arztpraxis die Nutzung der Infrastruktur vorübergehend zu erlauben, welche hierfür a priori kein Fachpersonal sind, um gegebenenfalls eine der Menge nach größtmögliche Versorgung der Bevölkerung mit den durch die betreffende Infrastruktur bereitgestellten Leistungen zu erreichen.

Die US 7 103 776 offenbart einen Notfall-Login-Vorgang in eine medizinische Infrastruktur, bei dem einem Nutzer insbesondere auch ohne Authentifizierung Zugang zur Infrastruktur gewährt werden kann.

Der Erfindung liegt daher die Aufgabe zugrunde, ein Verfahren zur Kontrolle der Nutzung einer medizinischen Infrastruktur anzugeben, welches für einen Notfall eine umfassendere Nutzung der Funktionalität der Infrastruktur als im Normalbetrieb vorgesehen erlaubt. Der Erfindung liegt weiter die Aufgabe zugrunde, eine medizinische Vorrichtung anzugeben, deren Nutzungsberechtigung sich in einem Notfall auf möglichst einfache Weise erweitern lässt.

Die erstgenannte Aufgabe wird erfindungsgemäß gelöst durch ein Verfahren nach Anspruch 1.

Die medizinische Infrastruktur kann hierbei insbesondere als Hardware-Infrastruktur oder als Software-Infrastruktur gegeben sein. Eine Hardware-Infrastruktur kann hierbei eines oder mehrere Geräte umfassen, wie sie insbesondere zur medizinischen Bildgebung und/oder zur medizinisch-biochemischen Analyse von Proben beispielsweise in einem Krankenhaus oder in einer spezialisierten Arztpraxis Anwendung finden. Als bildgebende medizinische Geräte sind hierbei insbesondere ein CT, ein Gerät zur Magnetresonanztomografie (MRT), ein C-Bogen-Röntgengerät, allgemein ein Gerät zur Angiografie und ein Gerät zur Abbildung von Körpergewebe mittels Ultraschall oder Röntgenstrahlung als medizinische Infrastruktur umfasst. Eine Software-Infrastruktur kann insbesondere durch ein spezifisches Programm zur Darstellung von medizinischen Bilddaten gegeben sein, welches beispielsweise einem behandelnden Radiologen oder sonstigem Fachpersonal ermöglicht, in CT- oder MRT-Bilddaten beliebige Schnittebenen durch das betreffende Volumenmodell des untersuchten Körpergewebes zu legen, und gegebenenfalls verschiedene Arten an Körpergewebe farblich unterschiedlich hervorzuheben, und insbesondere derart verarbeitete bzw. dargestellte Bilddaten auf einem Datenträger oder durch einen Ausdruck auszugeben. Hierbei sind auch Mischformen einer Hardware- und Software-Infrastruktur denkbar, bei welchen beispielsweise ein spezifisches Programm zur medizinischen Datenanalyse auf einer eigens hierfür optimierten Rechnereinheit läuft.

Als Nutzer-Ebene der medizinischen Infrastruktur kann hierbei insbesondere ein Krankenhaus oder auch eine einzelne medizinische Fachabteilung eines Krankenhauses, aber auch eine Arztpraxis, insbesondere eine Gemeinschaftspraxis innerhalb eines Praxisverbundes wie zum Beispiel einem Ärztehaus fungieren. Die Nutzer-Ebene kann, mit anderen Worten, insbesondere durch eine abgegrenzte bzw. abgrenzbare Institution gegeben sein, in welcher eine medizinische Diagnostik und/oder eine medizinische Behandlung wenigstens in ihren jeweils wesentlichen Schritten erfolgt, und welche sich für die besagte medizinische Diagnostik und/oder Behandlung der vorliegenden medizinischen Infrastruktur bedient. Eine Abgrenzung der Nutzer-Ebene kann hierbei einerseits rein räumlich erfolgen, sodass beispielsweise sämtliche einzelnen medizinischen Fachabteilungen eines räumlich zusammenhängenden Krankenhauskomplexes als eine gemeinsame Nutzer-Ebene definiert sein können. Die Abgrenzung kann aber auch anhand von rechtlichorganisatorischen Gesichtspunkten erfolgen, sodass beispielsweise einzelne, organisatorisch voneinander jeweils getrennte und insbesondere durch unterschiedliche Träger betriebene Einzel-Institutionen innerhalb eines größeren, räumlich zusammenhängenden Klinik-Verbunds jeweils als eigenständige Nutzer-Ebenen angesehen werden können. Demgegenüber ist die Hersteller-Ebene insbesondere als diejenige Sphäre der medizinischen Infrastruktur anzusehen, in welcher sämtliche Konstruktions- und/oder Produktionsschritte für die Infrastruktur erfolgen, und von welcher aus insbesondere Leistungen zur Wartung und/oder Instandhaltung der Infrastruktur erfolgen. Die Hersteller-Ebene kann hierbei insbesondere implementierte Maßnahmen zur Erfassung einer Nutzung der Infrastruktur (also Art und/oder Umfang der Nutzung) umfassen, welche insbesondere zum Zweck einer entsprechenden Verrechnung im Rahmen eines Lizensierungsmechanismus erfolgen kann.

Der Normalmodus ist hierbei insbesondere durch diejenigen Funktionen der Infrastruktur charakterisiert, welche der Nutzer-Ebene im Rahmen eines Lizensierungsmechanismus und/oder eines Vertrages über die Nutzung zwischen der Nutzer-Ebene und der Hersteller-Ebene grundsätzlich zur Verfügung gestellt werden. Mit anderen Worten kann also der Normalmodus die Nutzer-Ebene dazu befähigen, an der Infrastruktur genau die von der Nutzer-Ebene bei der Hersteller-Ebene gebuchten Funktionen zu nutzen. Eine derartige Nutzung erfolgt dann insbesondere im Rahmen der regulären Nutzungsberechtigung. Die reguläre Nutzungsberechtigung kann hierbei auf einen bestimmten Personenkreis innerhalb der Nutzer-Ebene eingeschränkt sein. Während auf der Nutzer-Ebene verschiedene Gruppen von allgemein medizinischem Personal agieren, also insbesondere Ärzte, Arzthelfer, medizinisch-technisches Personal, Krankenpfleger, etc., ist die reguläre Nutzungsberechtigung bevorzugt auf einen bestimmten Kreis von Personen aus diesen Gruppen, welche nachweislich über die nötigen Fachkenntnisse zur Nutzung der Infrastruktur verfügen, eingeschränkt. Hierdurch kann im Normalmodus, welcher den alltäglichen Betrieb der Infrastruktur auf der Nutzer-Ebene charakterisiert, eine unsachgemäße Nutzung, welche potenziell mit Risiken für Patienten und/oder Personal der Nutzer-Ebene verbunden sein können, vermieden werden. Insbesondere wird im Normalmodus eine Nutzung der Infrastruktur, welche durch die reguläre Nutzungsberechtigung erfolgt, auch anhand dieser erfasst, beispielsweise für eine Verrechnung mit der Hersteller-Ebene im Rahmen eines Lizensierungsmechanismus.

Unter einem Einrichten der regulären Nutzungsberechtigung auf der Hersteller-Ebene ist hierbei insbesondere zu verstehen, dass auf der Hersteller-Ebene bei der Konzeption bzw. der Konstruktion der Infrastruktur diejenigen Maßnahmen getroffen werden, welche die reguläre Nutzungsberechtigung in der Infrastruktur implementieren. Dass der konkrete Zugang im Rahmen der regulären Nutzungsberechtigung hierbei auf der Nutzer-Ebene noch durch das entsprechende Anlegen von ggf. personalisierten Benutzerkennungen geregelt sein kann, wird hiervon nicht berührt, da auch die grundsätzliche Funktionalität der Möglichkeit, derartige Benutzerkennungen einzurichten, auf der Hersteller-Ebene in der Infrastruktur vorgesehen und entsprechend eingerichtet wird.

Insbesondere ist im Normalmodus die Nutzung der Infrastruktur ausschließlich durch die reguläre Nutzungsberechtigung ermöglicht. Die Aktivierung des Ausnahme-Modus erfolgt bevorzugt durch einen lokalen Administrator auf der Nutzer-Ebene, dessen Befugnisse über die Infrastruktur hinaus reichen können, jedoch nicht über die Nutzer-Ebene hinaus reichen. Der lokale Administrator kann dabei beispielsweise durch einen Sicherheitsadministrator oder auch durch einen leitenden Mediziner im Krankenhaus oder in der Krankenhaus-Fachabteilung gegeben sein, welche die Nutzer-Ebene bildet. Bevorzugt kann die Ausnahme-Nutzungsberechtigung dergestalt sein, dass durch diese die Nutzung der Infrastruktur einem gegenüber der regulären Nutzungsberechtigung wesentlich erweiterten Personenkreis auf der Nutzer-Ebene ermöglicht wird. Während also bevorzugt die reguläre Nutzungsberechtigung nur einen eingeschränkten Personenkreis der auf der Nutzer-Ebene tätigen Mitarbeiter (also zum Beispiel Fachärzte und für die Infrastruktur spezifisch geschultes medizinisch-technisches Fachpersonal) für eine Nutzung der Infrastruktur vorsieht, und/oder nur eine konkret vorgegebenen Umfang an Funktionen der Infrastruktur zur Nutzung freigeschaltet, kann die Infrastruktur durch die Ausnahme-Nutzungsberechtigung bevorzugt durch eine einfache Benutzerkennung jedes beliebigen, auf der Nutzer-Ebene tätigen Mitarbeiters genutzt werden und/oder eine Nutzung sämtlicher von der Konstruktion bzw. Konzeption her möglichen Funktionen der Infrastruktur in beliebigem Umfang und in beliebiger Anzahl ermöglicht werden. Insbesondere kann hierbei der Ausnahme-Modus durch ein Einrichten bzw. ein Vorhandensein der Ausnahme-Nutzungsberechtigung bereits vollständig charakterisiert sein, d. h., der Ausnahme-Modus weist in diesem Fall keine über die Ausnahme-Nutzungsberechtigung hinausgehenden, weiteren Merkmale auf.

Unter einem Notfall ist hierbei insbesondere eine derartige Notsituation zu verstehen, in welcher aufgrund eines äußeren, insbesondere unvorhersehbaren Ereignisses mit einer erheblich gesteigerten Nachfrage für die Nutzung der Infrastruktur zu rechnen ist, also insbesondere eine Naturkatastrophe, eine Epidemie, oder auch ein großer terroristischer Angriff. Ist beispielsweise die Infrastruktur durch ein CT-Gerät gegeben, und die Nutzer-Ebene durch ein Krankenhaus, in welchem das CT-Gerät in einer Fachabteilung mit einer eingeschränkten Nutzung zum Zwecke der Diagnostik in besagter Fachabteilung aufgestellt ist und betrieben wird, und ereignet sich im Umfeld des Krankenhauses ein schweres Erdbeben mit einer Vielzahl an Häuser-Einstürzen, so ist davon auszugehen, dass in unmittelbarer Zukunft eine Vielzahl von Patienten mit Knochenbrüchen und/oder inneren Verletzungen zu behandeln sein wird. Für eine optimale Behandlung ist hierbei der Einsatz von einem CT-Gerät zur schnellen Diagnose erforderlich, sodass infolge des Erdbebens die Nachfrage für die Nutzung des vorhandenen CT-Geräts schlagartig ansteigt. In diesem Fall bildet das Erdbeben den Auslöser für den vorliegenden Notfall.

Als erste, den Ausnahme-Modus betreffende Information kann einerseits eine solche Information übermittelt werden, welche lediglich die Aktivierung als solche betrifft, also insbesondere, dass eine Aktivierung des Ausnahme-Modus stattgefunden hat, und gegebenenfalls zu welchem Zeitpunkt. Eine Übermittlung der ersten Information an die Hersteller-Ebene kann hierbei insbesondere durch E-Mail, einen Messenger-Service oder auch über ein ansonsten für Instandhaltung und Service-Dienstleistungen vorgesehenes Netzwerk zwischen der Hersteller-Ebene und der Nutzer-Ebene erfolgen. Als eine Kontrolleinrichtung der Nutzer-Ebene kann hierbei insbesondere eine zentrale Leitstelle eines Krankenhauses oder einer Krankenhaus-Fachabteilung fungieren, von welcher aus eine korrekte Funktionalität sowie gegebenenfalls eine korrekte Stromversorgung etc. für sämtliche auf der Nutzer-Ebene eingerichtete Hardware und/oder Software umfassen. Die erste Information kann jedoch insbesondere auch eine konkrete Nutzung der Infrastruktur anhand der Ausnahme-Nutzungsberechtigung betreffen, also insbesondere Zeitpunkt, Art und Umfang der Nutzung.

Die zweite Information kann inhaltlich insbesondere identisch zur ersten Information ausgestaltet sein, falls sowohl eine erste Information übermittelt wird, als auch eine zweite Information irreversibel gespeichert wird. Generell gilt für den Inhalt der zweiten Information insbesondere das hinsichtlich der ersten Information gesagte. Der Unterschied zwischen der ersten Information und der zweiten Information wird insbesondere lediglich durch den Zielort der Information bestimmt, also im Fall der ersten Information durch eine räumlich von der Infrastruktur getrennten Zielort, und im Fall der zweiten Information durch ein nicht-flüchtiges Speichermedium bevorzugt in einer unmittelbaren Umgebung der Infrastruktur.

Als ein nicht-flüchtiges Speichermedium ist hierbei insbesondere ein USB-Stick, eine CD/DVD-ROM oder auch ein spezifischer, ausschließlich für diesen Zweck vorgesehener Hardware-Dongle umfasst. Die zweite Information kann hierbei insbesondere in die von der Infrastruktur erzeugten und/oder verarbeiteten Daten in Form einer Signatur oder eines digitalen Wasserzeichens o.ä. eingearbeitet sein. In diesem Fall kann der nicht-flüchtige Speicher einerseits durch den internen Speicher der Infrastruktur gegeben sein, wenn es sich bei dieser um eine Hardware-Infrastruktur handelt, oder durch einen internen Speicher der Recheneinheit, auf welcher die als eine Software ausgestaltete Infrastruktur implementiert ist, andererseits kann der nicht-flüchtige Speicher zusätzlich zu den oben genannten Speichermedien auch als Papierausdruck der erzeugten Daten ausgebildet sein.

Der irreversible Speichervorgang der zweiten Information kann im Fall, dass die zweite Information in einem hierfür dedizierten Speicherbereich zu speichern ist, in diesen mittels einer sogenannten eFuse "eingebrannt" werden. Eine derartige eFuse umfasst einen Bit-Wert, welcher lediglich von 0 auf 1 gesetzt werden kann, wobei ein erneutes Zurücksetzen auf 0 im Programm nicht als Möglichkeit implementiert ist. Alternativ oder zusätzlich hierzu können bevorzugt die von der Infrastruktur erzeugten und/oder verarbeiteten Daten, welche in einer entsprechenden Ausgabedatei ausgegeben werden, mit der zweiten Information versehen werden, beispielsweise durch einen entsprechenden Header in einer elektronischen Datei und/oder durch ein Einprägen von digitalen Wasserzeichen oder auch sichtbaren Informationen in eine Ausgabedatei im Fall einer Bilddatei.

Insbesondere kann ein Notfall auch durch einen Hacker-Angriff auf die Rechnersysteme und -netzwerke der Nutzer-Ebene gegeben sein. In diesem Fall ist eine Verbindung der Rechnersysteme und -netzwerke der Nutzer-Ebene mit der Außenwelt sofort zu trennen. Eine derartige Trennung kann jedoch ggf. auch die Verbindung zu einem Lizenz-Server auf der Hersteller-Ebene betreffen, welcher im Rahmen eines Lizensierungsmechanismus die Nutzung der Infrastruktur erfasst und entsprechend freischaltet. Im Fall eines Hacker-Angriffs mit den genannten Konsequenzen kann die Infrastruktur mit der Ausnahme-Nutzungsberechtigung weiterbetrieben werden.

Die vom Verfahren vorgeschlagenen Maßnahmen ermöglichen es, in einem Notfall durch die Ausnahme-Nutzungsberechtigung die Infrastruktur in einem möglichst uneingeschränkten, bevorzugt völlig uneingeschränkten Rahmen zu nutzen, wobei durch die erste bzw. die zweite Information ein Missbrauch der Aktivierung des Ausnahme-Modus unterbunden werden kann. So schützt die Übermittlung der ersten Information an die Kontrolleinrichtung auf der Nutzer-Ebene letztere gegen eine unberechtigte Aktivierung des Ausnahme-Modus, weil dieser und/oder eine entsprechende Nutzung sofort durch die Kontrolleinrichtung erkannt werden können. Eine missbräuchliche Aktivierung des Ausnahme-Modus mit dem Ziel einer Umgehung von Beschränkungen durch die Nutzer-Ebene kann einerseits, falls kein Notfall vorliegt, infolge der Übermittlung der ersten Information auf der Hersteller-Ebene entsprechend erfasst werden. Die Hersteller-Ebene ist bei einer absichtlichen und somit missbräuchlichen Blockierung einer Übermittlung der ersten Information durch die Möglichkeit einer nachträglichen Kontrolle anhand der zweiten Information zusätzlich gegen einen Missbrauch durch die Nutzer-Ebene geschützt.

Anhand von Daten über einzelne Nutzungsvorgänge, welche als zweite Information irreversibel gespeichert werden, besteht auf der Hersteller-Ebene zudem eine Möglichkeit einer nachträglichen Abrechnung der Nutzung, welche durch die Ausnahme.-Nutzungsberechtigung erfolgt ist, wenn die Aktivierung des Ausnahme-Modus tatsächlich infolge eines Notfalls vorgenommen wurde, und hierbei insbesondere eine Übermittlung der ersten Information an die Hersteller-Ebene infolge von notfallbedingt nicht existierenden Verbindungen zur Übermittlung ausgeblieben ist.

Bevorzugt werden Daten, welche die Aktivierung des Ausnahme-Modus betreffen, als erste Information übermittelt und/oder als zweite Information gespeichert. Insbesondere können diese Daten die Aktivierung unabhängig von einer tatsächlichen Nutzung der Infrastruktur mittels der Ausnahme-Nutzungsberechtigung charakterisieren, also beispielsweise allgemein Informationen wie einen Zeitpunkt der Aktivierung und ggf. einen Ort der Durchführung der Aktivierung, sowie ggf. eine Kennung eines durchführenden Administrators o.ä. Ein Übermitteln einer derartigen ersten Information an die Kontrolleinrichtung der Nutzer-Ebene erlaubt es dieser, eine missbräuchliche Aktivierung umgehend zu erkennen und ggf. entsprechende Sicherheitsmaßnahmen wie z.B. räumliche Zugangsbeschränkungen zu bestimmten medizinischen Abteilungen und/oder zur Infrastruktur vorzunehmen. Die Hersteller-Ebene kann durch die erste Information dazu veranlasst werden, ggf. Instandhaltungsmaßnahmen oder sonstige den Betrieb der Infrastruktur betreffenden Fern-Dienstleistungen daran anzupassen, dass ein internes Service-Netzwerk zwischen der Hersteller-Ebene und der Nutzer-Ebene vorübergehend nicht mehr zur Verfügung stehen könnte.

Zweckmäßigerweise wird ein durch die Ausnahme-Nutzungsberechtigung erfolgter Nutzungsvorgang der Infrastruktur erfasst, wobei Daten über den derart erfassten Nutzungsvorgang im Ausnahme-Modus als erste Information übermittelt und/oder als zweite Information gespeichert werden. Die Daten können hierbei insbesondere einen Start- und Endzeitpunkt des einzelnen Nutzungsvorganges, sowie ggf. eine Nutzerkennung, und weiter auch den Nutzungsumfang der Funktionen wie z.B. Anzahl der erzeugten Bilder in Fall eines bildgebenden medizinischen Gerätes als Infrastruktur umfassen. Eine bevorzugt unmittelbar bei ihrer Erfassung erfolgende Übermittlung derartiger Daten an die Hersteller-Ebene und/oder eine irreversible Speicherung ermöglichen der Hersteller-Ebene insbesondere eine nachträgliche Verrechnung der so erfolgten Nutzung.

Bevorzugt wird ein im Normalmodus durch die reguläre Nutzungsberechtigung erfolgender Nutzungsvorgang erfasst, und eine dritte Information über den derart erfassten Nutzungsvorgang im Normalmodus an die Herstellerebene übermittelt. Dies bedeutet, dass im Normalmodus jeder einzelne Nutzungsvorgang generell an die Hersteller-Ebene übermittelt, beispielsweise an ein zentrales Nutzungsprotokoll für die Infrastruktur auf einem entsprechenden Server. Anhand dieser dritten Information kann eine Abrechnung der Nutzung durch die Hersteller-Ebene an die Nutzer-Ebene erfolgen, bzw. eine Nutzung einer Teilfunktionalität freigeschaltet werden.

Als weiter vorteilhaft erweist es sich, wenn die reguläre Nutzungsberechtigung für eine Nutzung der Infrastruktur im Normalmodus durch einen ersten Lizensierungsmechanismus implementiert wird. Unter einem Lizensierungsmechanismus ist hierbei insbesondere die Gesamtheit aller Maßnahmen zu verstehen, welche die Nutzung im Rahmen der jeweils vorliegenden Nutzungsberechtigung ermöglichen, also insbesondere eine Lizenz in Form einer entsprechenden Vereinbarung zwischen der Hersteller-Ebene und der Nutzer-Ebene, eine Erfassung und Zuordnung der Nutzung der Infrastruktur auf der Nutzer-Ebene, ein Ausschluss unberechtigter Nutzung durch entsprechende Sicherheitsmaßnahmen, aber auch eine ggf. kryptografisch gesicherte Kommunikation zwischen der Infrastruktur und einem Lizenz-Server auf der Hersteller-Ebene. Eine derartige Implementierung eines Lizensierungsmechanismus wird heute oftmals bei medizinischer Infrastruktur angewandt, indem jeder einzelne Nutzungsvorgang zur Verrechnung bzw. auch zur einzelnen Freischaltung teilweise erfasst wird.

Günstigerweise wird die erste Information insbesondere für eine Übermittlung von der Infrastruktur an die Hersteller-Ebene über ein Service-Netzwerk übermittelt, welches bevorzugt zur Hersteller-seitigen Instandhaltung und/oder Funktionskontrolle etc. der Infrastruktur vorgesehen und eingerichtet ist. Hierdurch können bereits für andere Zwecke vorhandene Übertragungskanäle genutzt werden, wodurch die Implementierung eigener Übertragungskanäle zum Zweck einer Kontrolle der Nutzung unterbleiben kann.

In einer vorteilhaften Ausgestaltung der Erfindung wird zur Aktivierung des Ausnahme-Modus ein Aktivierungs-Datenschlüssel erzeugt und auf einem zweiten nicht-flüchtigen Speichermedium hinterlegt, wobei die Aktivierung des Ausnahme-Modus anhand des zweiten nicht-flüchtigen Speichermediums erfolgt. Das zweite nicht-flüchtige Speichermedium kann hierbei insbesondere durch einen physikalischen Speicher oder Datenträger gegeben sein, oder auch als eine Karte mit einem optisch auszulesenden QR-Code implementiert sein, wobei der zweite nicht-flüchtige Speicher auf der Nutzer-Ebene bevorzugt derart gesichert aufbewahrt wird, dass nur ein sehr begrenzter Kreis an Administratoren Zugriff darauf hat, wobei als Administrator auch ein Abteilungsleiter wie z.B. ein Chefarzt o.ä. agieren kann.

Als weiter vorteilhaft erweist es sich, wenn das erste nicht-flüchtige Speichermedium in der Infrastruktur eingerichtet, und/oder das erste nicht-flüchtige Speichermedium für eine physikalische Verbindung mit der Infrastruktur vorgesehen und eingerichtet ist, und wenn weiter die zweite Information mittels wenigstens eines nicht rücksetzbaren Variablenwertes im ersten nicht-flüchtigen Speichermedium, z.B. insbesondere als "eFuse", gespeichert wird. Das erste nicht-flüchtige Speichermedium kann hierbei insbesondere mit dem zweiten nicht-flüchtigen Speichermedium identisch sein, also beispielsweise als "Notfall-USB-Stick/Dongle", auf welchem ein Aktivierungs-Datenschlüssel zur Aktivierung des Ausnahme-Modus' schreibgeschützt hinterlegt ist. Das erste nicht-flüchtige Speichermedium kann innerhalb eines Netzwerkes auf der Nutzer-Ebene und/oder, im Fall einer Hardware-Infrastruktur, direkt in der Infrastruktur selbst implementiert sein. Bevorzugt führt im Ausnahme-Modus die Infrastruktur eine automatische Speicher-Routine für die zweite Information durch, wobei die jeweils erzeugten Daten der zweiten Information schreibgeschützt, z.B. als Protokoll, Blockchain etc. gespeichert werden. Hierdurch kann ein Löschen der zweiten Information wirksam unterbunden werden, wodurch ein missbräuchliches Aktivieren des Ausnahme-Modus' zur Umgehung der Beschränkungen der regulären Nutzungsberechtigung durch die Nutzer-Ebene nachträglich für die Hersteller-Ebene erkannt werden kann.

Zweckmäßigerweise werden bei der Nutzung der Infrastruktur eine Mehrzahl an medizinischen Nutz-Informationen erzeugt und/oder verarbeitet, wobei die medizinischen Nutz-Informationen in einer Anzahl an Ausgabedateien gespeichert werden, wobei im Ausnahme-Modus die zweite Information in einer Ausgabedatei, bevorzugt in jeder der im Ausnahme-Modus erzeugten Ausgabedateien, gespeichert wird. Dies kann z.B. in einem Header der Ausgabedatei erfolgen. Hierdurch lässt sich eine Nutzung der Infrastruktur durch die Ausnahme-Nutzungsberechtigung auch anhand der als Ergebnis der Nutzung erzeugten Daten dokumentieren, was einen Nachweis einer berechtigten Nutzung und auch eines möglichen Missbrauchs erleichtert.

Bevorzugt wird im Ausnahme-Modus die betreffende Ausgabedatei mit einer digitalen Signatur versehen, wobei die digitale Signatur anhand der jeweiligen medizinischen Nutz-Informationen der Ausgabedatei erzeugt wird, welche bei der durch die Ausnahme-Nutzungsberechtigung erfolgenden Nutzung der Infrastruktur erzeugt und/oder verarbeitet wurden. Bevorzugt wird auch im Normalmodus eine Ausgabedatei mit einer auf vergleichbare Art gewonnenen digitalen Signatur versehen. Insbesondere erfolgt die Erzeugung der digitalen Signatur anhand einer Hashfunktion oder eines ähnlichen kryptografischen Algorithmus', welcher von Dritten und somit auch von der Nutzer-Ebene nicht invertiert werden kann. Hierdurch kann eine nachträgliche Manipulation der Ausgabedatei zum Zweck einer Verschleierung der zweiten Information vermieden oder zumindest erkannt werden, da durch die Manipulation die Signatur nicht mehr zu den Nutz-Informationen kompatibel ist.

Vorteilhafterweise wird als medizinische Infrastruktur ein bildgebendes medizinisches Gerät genutzt, welches dazu eingerichtet ist, von einer Körperregion eines Patienten mittels einer medizinischen Abbildungsmodalität Bilddaten zu erzeugen, oder als medizinische Infrastruktur wird eine medizinische Bilddarstellungs-Software genutzt, mittels derer von einem bildgebenden medizinischen Gerät erzeugte Bilddaten auf einer entsprechend eingerichteten Rechnereinheit darstellbar und/oder verarbeitbar sind. Gerade für die genannten Formen der medizinischen Infrastruktur aus dem Bereich der medizinischen Bildgebung ist das vorgeschlagene Verfahren infolge der für die genannten Infrastrukturen vielfältigen Abstufungsmöglichkeiten ihrer Nutzung vorteilhaft.

Bevorzugt wird hierbei die zweite Information als eine Bildinformation in den erzeugten bzw. verarbeiteten Bilddaten gespeichert, also insbesondere in unsichtbarer Form, z.B. als digitales Wasserzeichen, und/oder in sichtbarer Form. Dies ermöglicht es, die Nutzung der Infrastruktur anhand der Ausnahme-Nutzungsberechtigung auch dann noch zu dokumentieren, wenn die ursprünglich erzeugten Bilddaten in ggf. komprimierter und/oder skalierter Form ausgedruckt und neu eingescannt oder als Screenshots auf einem Bildschirm abgespeichert werden.

Die zweitgenannte Aufgabe wird erfindungsgemäß gelöst durch eine medizinische Vorrichtung nach Anspruch 12.

Die medizinische Vorrichtung ist hierbei insbesondere durch ein bildgebendes medizinisches Gerät gegeben, welches Bilddaten als die medizinischen Nutz-Informationen anhand einer menschlichen Körperregion erzeugt, oder durch eine medizinisch-biochemische Analysevorrichtung, welche als die medizinischen Nutz-Informationen anhand von menschlichem Körpergewebe, Ausscheidungen oder Blutproben Analysedaten über die Beschaffenheit der Proben erzeugt. Die medizinische Vorrichtung ist bevorzugt zur Aktivierung des Ausnahme-Modus über einen Hardware-Dongle oder über einen Aktivierungs-Datenschlüssel auf entsprechendem Speichermedium eingerichtet, wenn der Hardware-Dongle bzw. das Speichermedium mit der Vorrichtung verbunden wird. Insbesondere ist die medizinische Vorrichtung zum irreversiblen Speichern der zweiten Information dahingehend eingerichtet, dass in der Vorrichtung in einem eigens dafür vorgesehenem Speicher oder einem dedizierten Speicherbereich der medizinischen Vorrichtung eine entsprechende Routine hinterlegt ist, welche die zweite Information vorzugsweise automatisch, also insbesondere ohne Nachfrage und/oder weitere Eingriffsmöglichkeit von außen, und schreibgeschützt gegen nachträgliche Eingriffe im ersten nicht-flüchtigen Speicher bzw. im physischen Speichermedium, welches an der zweiten Datenstelle verbunden ist, speichert.

Bevorzugt ist die medizinische Vorrichtung hierbei ausgebildet als ein bildgebendes medizinisches Gerät, also insbesondere al ein CT, MRT, Röntgen, oder C-Bogen-Angiograf etc., welches bei der Nutzung als medizinische Nutz-Informationen Bilddaten einer menschlichen Körperregion erzeugt, wobei die medizinische Vorrichtung dazu eingerichtet ist, erzeugte Bilddaten über eine zweite Datenschnittstelle auszugeben und/oder auf einem nicht-flüchtigen Speichermedium zu speichern, und wobei die medizinische Vorrichtung ferner dazu eingerichtet ist, die zweite Information als Bildinformation in den Bilddaten zu speichern. In diesem Fall kann auch das nicht-flüchtige Speichermedium als erster nicht-flüchtiger Speicher fungieren.

Die Erfindung nennt weiter ein Computerprogrammprodukt nach Anspruch 14. Das Computerprogrammprodukt kann Programmcode zur Durchführung einer Verfahrensroutine aufweisen, wenn das Computerprogrammprodukt auf einem Rechner ausgeführt wird, wobei die Verfahrensroutine eine Nutzung einer medizinischen Infrastruktur kontrolliert, wobei gemäß der Verfahrensroutine ein Normalmodus für die Nutzung der Infrastruktur über eine vorgegebene reguläre Zugangsberechtigung eingerichtet ist, ein Ausnahme-Modus aktivierbar ist, in welchem die Nutzung der Infrastruktur auch über eine Ausnahme-Zugangsberechtigung eingerichtet ist, wobei bei aktiviertem Ausnahme-Modus eine den Ausnahme-Modus betreffende erste Information über eine Programmschnittstelle zur Übermittlung durch den Rechner ausgegeben wird, und/oder eine den Ausnahme-Modus betreffende zweite Information zum irreversiblen Speichern, insbesondere auch zum Speichern in Ausgabedateien mit medizinischen Nutz-Informationen, ausgegeben wird. Die für das Verfahren und für seine Weiterbildungen angegebenen Vorteile können hierbei sinngemäß auf das Computerprogrammprodukt übertragen werden. Das besagte Computerprogrammprodukt kann hierbei einerseits die Nutzung einer Hardware-implementierten medizinischen Infrastruktur steuern und kontrollieren, eignet sich aber insbesondere aufgrund der Ausführung auf einem Rechner auch zur Kontrolle des Zugangs zu einer rein als Software implementierten medizinischen Infrastruktur. Die Kontrolle über die Nutzung kann hierbei insbesondere lokal erfolgen, indem z.B. die Software der Infrastruktur auf demselben Rechner ausgeführt wird wie das Computerprogrammprodukt. Die von der Verfahrensroutine kontrollierte Nutzung kann aber insbesondere auch Cloud-basiert, also von einem von der Infrastruktur räumlich und strukturell getrennten Rechner, erfasst werden.

Nachfolgend wird ein Ausführungsbeispiel der Erfindung anhand von Zeichnungen näher erläutert. Hierbei zeigen jeweils schematisch:
- FIG 1: in einem Blockdiagramm eine Nutzung eines bildgebenden medizinischen Geräts in einem Normalmodus,
- FIG 2: in einem Blockdiagramm die Nutzung des bildgebenden medizinischen Gerätes nach FIG 1 in einem Ausnahme-Modus, und
- FIG 3: in einem Blockdiagramm einen Nutzungsvorgang des bildgebenden medizinischen Gerätes im Ausnahme-Modus nach FIG 2.

Einander entsprechende Teile und Größen sind in allen Figuren jeweils mit gleichen Bezugszeichen versehen.

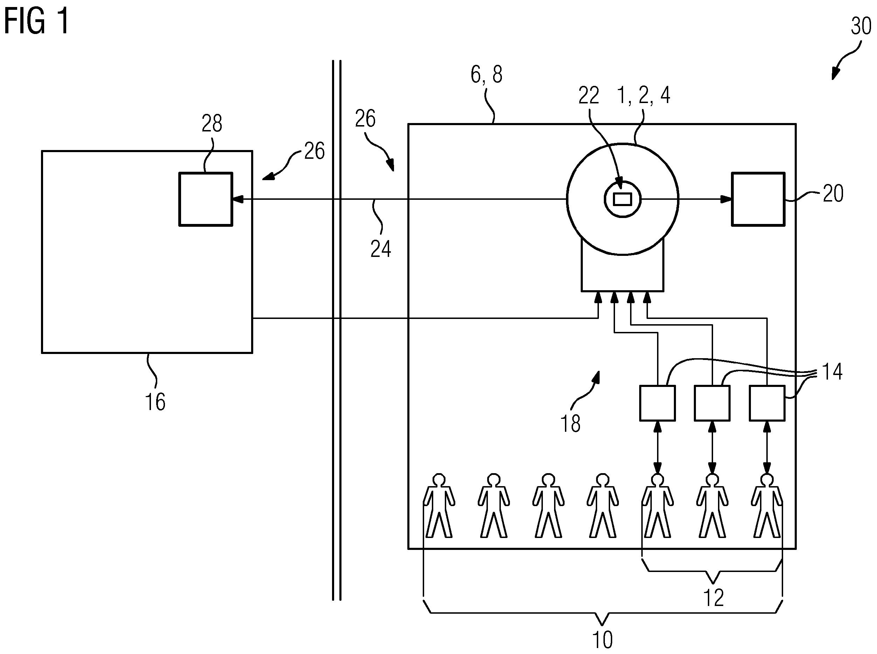

In FIG 1 ist schematisch in einem Blockdiagramm ein Verfahren dargestellt, mittels dessen die Nutzung einer medizinischen Infrastruktur 1 kontrolliert wird. Die medizinische Infrastruktur 1 ist vorliegend gegeben durch eine medizinische Vorrichtung 2, welche als ein bildgebendes medizinisches Gerät 4 ausgebildet ist. Das bildgebende medizinische Gerät 4 kann dabei beispielsweise durch einen CT, einen MRT oder auch einen C-Bogen-Angiografen gegeben sein, wobei diese Aufzählung keinesfalls als Beschränkung aufzufassen ist. Das bildgebende medizinische Gerät 4 ist hierbei in einem Krankenhaus 6 aufgestellt, welches zugleich für eine Nutzung des bildgebenden medizinischen Gerätes 4 die Nutzer-Ebene 8 bildet. Im Krankenhaus 6 ist eine erste Personengruppe 10 tätig, welche somit prinzipiell Zugang zum bildgebenden medizinischen Gerät 4 hat. Die erste Personengruppe 10 ist dabei insbesondere als die Gesamtheit aller im Krankenhaus 6 tätigen Personen aufzufassen, welche potenziell einen Zugriff auf das bildgebende medizinische Gerät 4 erhalten können (und nicht durch physische Schutzmechanismen wie zum Beispiel zugangsbeschränkte Türen zwischen einzelnen Fachabteilungen des Krankenhauses 6 daran gehindert sind). Die erste Personengruppe umfasst somit insbesondere die Ärzte, das medizinisch-technische Personal und gegebenenfalls noch Pflegepersonal des Krankenhauses 6.

Innerhalb der ersten Personengruppe 10 ist nun eine zweite Personengruppe 12 definiert, deren Personen einerseits durch ihre medizinische Ausbildung dazu befähigt sind, das bildgebende medizinische Gerät 4 ordnungs- und sachgemäß zu bedienen, und deren Tätigkeit im Krankenhaus 6 eine derartige Bedienung des bildgebenden medizinischen Gerätes 4 zumindest zeitweise erforderlich macht. Dies kann beispielsweise für spezialisierte Radiologen und/oder für ein auf die Anwendung des bildgebenden medizinischen Gerätes 4 spezialisiertes, medizinisch-technisches Fachpersonal gelten. Für die Personen der zweiten Personengruppe 12 sind hierbei jeweils insbesondere gerätespezifische Benutzerkennungen 14 eingerichtet, welche eine Nutzung des bildgebenden medizinischen Gerätes 4 erlauben. Für die anderen Personen der ersten Personengruppe 10, welche nicht zugleich der zweiten Personengruppe 12 angehören, ist die Nutzung des bildgebenden medizinischen Gerätes 4 nicht ermöglicht. Das konkrete Einrichten der Benutzerkennung und 14, also das jeweils individuelle Zuweisen einer Zugangsberechtigung der Nutzung zu einer Person der zweiten Personengruppe 12, kann dabei lokal auf der Nutzer-Ebene 8 (beispielsweise durch einen entsprechenden System-Administrator des Krankenhauses 6) erfolgen. Die grundsätzliche Möglichkeit, derartige Benutzerkennungen 14 einzurichten, ist dabei bereits durch den Hersteller des bildgebenden medizinischen Gerätes 4 in selbiges implementiert. Der Hersteller kann dabei gegebenenfalls dem Krankenhaus 6 (und insbesondere einem dortigen System-Administrator) entsprechende Hilfsmittel für das Einrichten zur Verfügung stellen.

Die zweite Personengruppe 12, kann nun eine vorab zwischen dem Krankenhaus 6 als Nutzer-Ebene 8 und einer Hersteller-Ebene 16 vereinbarten Anteil an den Funktionen des bildgebenden medizinischen Gerätes 4 nutzen. Die auf besagten Anteil beschränkte Nutzung ist in FIG 1 durch eine entsprechende Schraffur eines Teils des bildgebenden medizinischen Gerätes 4 angedeutet. Durch die in der beschriebenen Weise eingeschränkte Nutzungsmöglichkeit des bildgebenden medizinischen Gerätes 4 infolge der besagten Vereinbarungen zwischen der Hersteller-Ebene 16 und der Nutzer-Ebene 8 und/oder durch die Benutzerkennungen 14 für die Personen der zweiten Personengruppe 12 ist nun eine reguläre Nutzungsberechtigung 18 für eine Nutzung des bildgebenden medizinischen Gerätes 4 implementiert. Die reguläre Nutzungsberechtigung 18 kann hierbei einerseits den Ausschluss der nicht berechtigten Personen der ersten Personengruppe 10 von der Nutzung umfassen, andererseits auch die Einschränkung des vollen Funktionsumfang des bildgebenden medizinischen Gerätes 4 auf eine gemäß der besagten Vereinbarung zwischen der Hersteller-Ebene 18 und der Nutzer-Ebene 8 getroffenen Vereinbarung.

In einem einzelnen Nutzungsvorgang 22 des bildgebenden medizinischen Gerätes 4 wird nun ein Körperteil eines nicht näher dargestellten Patienten durch das bildgebende medizinische Gerät 4 gemäß dessen Abbildungsmodalität entsprechend in Bilddaten 20 umgesetzt. Dieser einzelne Nutzungsvorgang 22, bei welchem die Bedienung des bildgebenden medizinischen Gerätes 4 durch eine Person der zweiten Personengruppe 12 im Rahmen der regulären Nutzungsberechtigung 18 erfolgt, wird nun an die Hersteller-Ebene 16 gemeldet. Hierfür wird eine entsprechende Information 24 mit relevanten Daten des Nutzungsvorgangs 22, also beispielsweise Zeitpunkt und Dauer sowie Benutzerkennung 14 der bedienenden Person aus der zweiten Personengruppe 12, im Rahmen eines Lizensierungsmechanismus 26 vom bildgebenden medizinischen Gerät, 4 und somit von der Nutzer-Ebene 8 an eine Lizenz-Server 28 auf der Hersteller-Ebene 16 übertragen.

Die im Rahmen des Nutzungsvorgang 22 erzeugten Bilddaten 20 können zusätzlich ebenfalls mit den Daten der Information 24 versehen sein, zum Beispiel als Header der oder jeder Bilddatei und/oder als eine digitale Signatur, welche insbesondere von den Bilddaten selbst abhängen kann. Die in FIG 1 dargestellte Nutzung der medizinischen Infrastruktur 1 mittels der regulären Nutzungsberechtigung 18 ist hierbei insbesondere als diejenige Art der Nutzung anzusehen, welche im Normalfall und somit insbesondere ohne außergewöhnliche Vorkommnisse erfolgt. Mithin ist diese Art der Nutzung somit charakteristisch für einen Normalmodus 30 des bildgebenden medizinischen Gerätes 4.

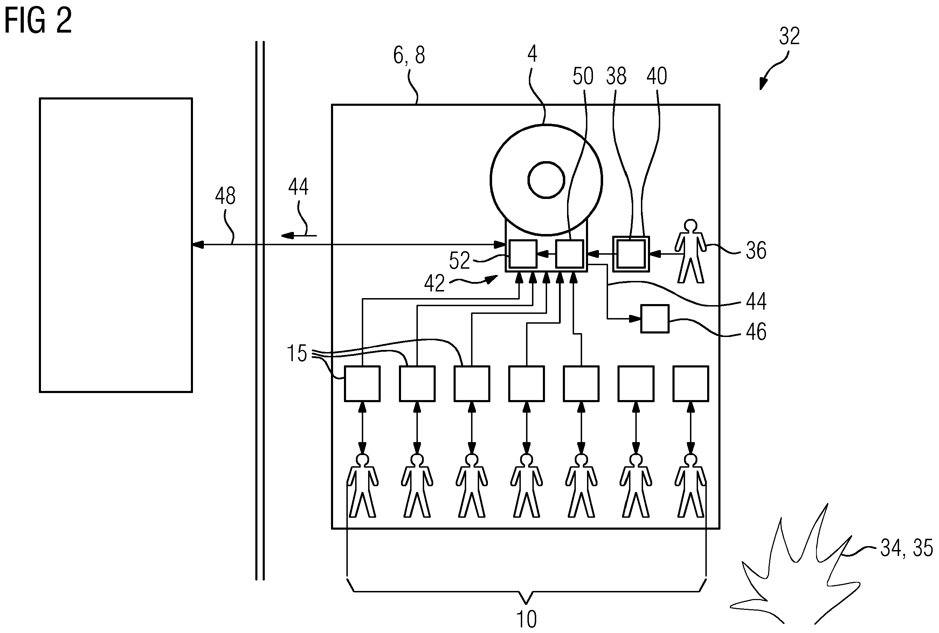

In FIG 2 ist nun schematisch in einem Blockdiagramm die Nutzung des bildgebenden medizinischen Gerätes 4 nach FIG 1 in einem Ausnahme-Modus 32 dargestellt. In räumlicher Nähe zum Krankenhaus 6 ereignet sich eine schwere Explosion 34, infolge derer eine Vielzahl an Menschen Verletzungen wie zum Beispiel Knochenbrüche oder auch potenziell innere Verletzungen erleiden. Zur bestmöglichen Behandlung der besagten Verletzungen sind nun bevorzugt alle in der Umgebung der Explosion 34 verfügbaren medizinischen Kapazitäten für eine unmittelbare Versorgung auszuschöpfen. Die Nachfrage nach der Nutzung des bildgebenden medizinischen Gerätes 4 im Krankenhaus 6 steigt erheblich an, und kann insbesondere im Rahmen der durch die reguläre Nutzungsberechtigung 18 vorhandenen Beschränkungen der Nutzung (einerseits hinsichtlich der Funktionalität und andererseits hinsichtlich der bedienenden Personen aus der zweiten Personengruppe 12) nicht vollständig bewältigt werden kann. Somit ist durch die Explosion 34 ein Notfall 35 gegeben, in welchem der Ausnahme-Modus 32 für eine vom in FIG 1 dargestellten Normalmodus 30 abweichende Nutzung des bildgebenden medizinischen Gerätes 4 ermöglicht wird.

Ein Sicherheitsadministrator 36 überträgt einen Aktivierungs-Datenschlüssel 38, welcher auf einem Speichermedium 40 hinterlegt ist, auf das bildgebende medizinische Gerät 4, wodurch der Ausnahme-Modus 32 unmittelbar aktiviert wird, und infolgedessen eine Ausnahme-Nutzungsberechtigung 42 für die Nutzung des bildgebenden medizinischen Gerätes 4 freigeschaltet wird. Die Ausnahme-Nutzungsberechtigung 42 erlaubt es nun allen Personen der ersten Personengruppe 10, für die Dauer des Ausnahme-Modus 32 das bildgebende medizinische Gerät 4 mittels ihrer jeweiligen, auf der Nutzer-Ebene 6 gültigen Benutzerkennung 15 im vollen Funktionsumfang nutzen zu können. Das Speichermedium 40 kann hierbei beispielsweise durch einen USB-Stick oder eine Hardware-Dongle gegeben sein. In diesem Fall ist der Aktivierung-Datenschlüssel 38 durch eine entsprechende Datei auf dem Speichermedium 40 gegeben, welche zur Aktivierung des Ausnahme-Modus 32 vom bildgebenden medizinischen Gerät 4 bei einer Verbindung des Speichermediums 40 sofort und automatisch ausgeführt wird. Alternativ kann der Aktivierungs-Datenschlüssel in einem QR-Code bestehen, welcher auf einer Karte als Speichermedium 40 aufgedruckt ist. Das Übertragen des Aktivierungs-Datenschlüssels 38 auf das bildgebende medizinische Gerät 4 erfolgt dann durch eine entsprechende optische Erfassung. Als eine weitere Alternative zum Aktivierungs-Datenschlüssel 38, oder aber auch zusätzlich zu diesem kann ein physikalischer Schließmechanismus mit einem entsprechenden Schlüssel zur Aktivierung des Ausnahme-Modus 32 am bildgebenden medizinischen Gerät 4 vorgesehen sein. Bevorzugt befindet sich das Speichermedium 40 im Krankenhaus 6 bzw. auf der Nutzer-Ebene 8 derart unter Verschluss, dass lediglich der Sicherheitsadministrator 36 (hierbei können auch mehrere Sicherheitsadministrator 36 vorgesehen sein) Zugriff darauf hat.

Als eine unmittelbare Konsequenz der Aktivierung des Ausnahme-Modus 32 wird sofort eine erste Information 44 über die Aktivierung, bevorzugt zusammen mit Daten zum Zeitpunkt der Aktivierung sowie zur Identität des Sicherheitsadministrators 36, welcher die Aktivierung durchgeführt hat, an die Hersteller-Ebene 16 sowie an eine Kontrolleinrichtung 46 auf der Nutzer-Ebene 8 übertragen. Die Kontrolleinrichtung 46 kann hierbei beispielsweise durch eine interne Leitstelle des Krankenhauses 6 gegeben sein, in welcher der ordnungsgemäße Betrieb sämtlicher Installationen überwacht wird. Anhand der ersten Information 44 kann in der Kontrolleinrichtung 46 der Nutzer-Ebene 8 eine missbräuchliche Aktivierung des Ausnahme-Modus 32, wenn kein Notfall 35 vorliegt, sofort erkannt werden, sodass zusätzliche Sicherheitsmaßnahmen wie z.B. das Sperren einzelner Krankenhaus-Trakte ergriffen werden können.

Für eine Übertragung der ersten Information 44 an die Hersteller-Ebene kann beispielsweise ein zwischen der Hersteller-Ebene 16 und der Nutzer-Ebene 8 vorexistierendes Service-Netzwerk 48 genutzt werden, welches ansonsten durch die Hersteller-Ebene für Instandhaltungsmaßnahmen oder zur Fehlerbehebung des bildgebenden medizinischen Gerätes 4 genutzt wird. Alternativ oder auch zusätzlich hierzu kann die erste Information 44 über die Aktivierung des Ausnahme-Modus 32 auch über die im Rahmen des Lizensierungsmechanismus 26 implementierten Kommunikationskanäle zwischen der Hersteller-Ebene 16 und der Nutzer-Ebene 8 übertragen werden. Des Weiteren wird eine zweite Information 50 über die Aktivierung irreversibel in einen nicht-flüchtigen Speicher 52 des bildgebenden medizinischen Gerätes 4 gespeichert. Hierbei kann beispielsweise auf einer Betriebssystem-, oder BIOS-Ebene des bildgebenden medizinischen Gerätes 4 ein Speichern der zweiten Information 50 derart eingerichtet werden, dass eine nachträgliche Bearbeitung oder gar Löschung der einmalig gespeicherten zweiten Information 50 nicht realisierbar ist.

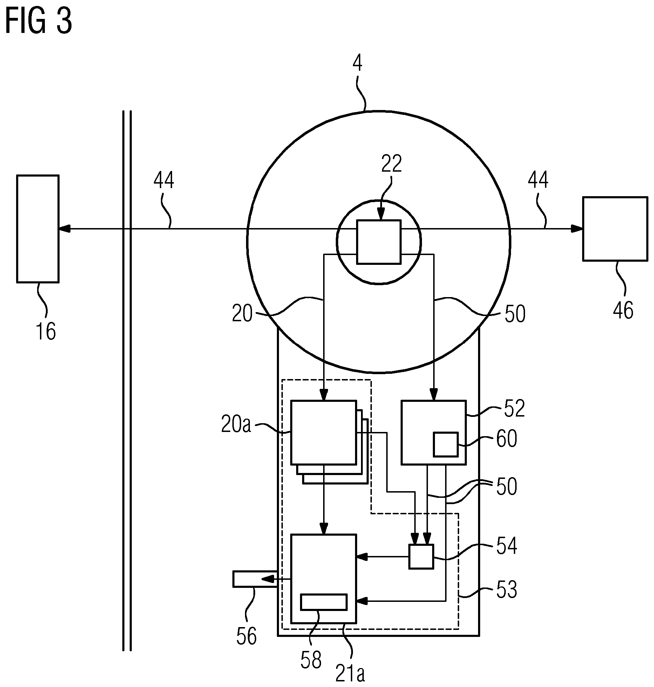

In FIG 3 ist schematisch in einem Blockdiagramm ein Nutzungsvorgang 22 des bildgebenden medizinischen Gerätes 4 dargestellt, welcher im Ausnahme-Modus 32 durchgeführt wird. Eine nicht näher dargestellte Körperregion eines Patienten wird durch das bildgebende medizinische Gerät 4 gemäß dessen Abbildungsmodalität in Bilddaten 20 umgesetzt. Dese Bilddaten liegen zunächst nur in einem möglicherweise flüchtigen Speicher 53 des bildgebenden medizinischen Gerätes 4 vor. Zu Beginn und/oder während des Nutzungsvorgangs 22, also vorliegend der Bildaufnahme, und/oder unmittelbar nach dessen Beendigung werden vom bildgebenden medizinischen Gerät 4 als erste Information 44 Daten bezüglich des Nutzungsvorgangs 22 an die Kontrolleinheit 46 und/oder an die Hersteller-Ebene 16 übermittelt. Diese Daten umfassen insbesondere Start- und ggf. Endzeitpunkt der Nutzung sowie den konkret genutzten Funktionenumfang des bildgebenden medizinischen Gerätes 4, zudem die Ausnahme-Nutzungsberechtigung 42 und ggf. die Benutzerkennung 15 der bedienenden Person.

Liegt kein Notfall 35 vor, und ist insbesondere die Verbindung zur Hersteller-Ebene 16 über das Service-Netzwerk 48 intakt, so kann die Hersteller-Ebene 16 die Nutzung anhand der Ausnahme-Nutzungsberechtigung 42 als solche erkennen, und ggf. eine Anfrage an die Nutzer-Ebene über den Grund der erfolgten Aktivierung des Ausnahme-Modus 32 stellen, um einerseits einer Aktivierung vorzubeugen, welche auf Missbrauch oder auch auf Fehleinschätzungen der tatsächlichen Lage beruht, und um andererseits im Fall eines Notfalls 35 ggf. zusätzliches Wartungspersonal etc. abstellen und zum Krankenhaus 6 beordern zu können, und so im Notfall 35 einen möglichst reibungslosen Betrieb des bildgebenden medizinischen Geräts 4 gewährleisten zu können.

Zu Beginn und/oder während des Nutzungsvorgangs 22 und/oder unmittelbar nach dessen Beendigung werden vom bildgebenden medizinischen Gerät 4 überdies als zweite Information 50 die zur ersten Information 44 identischen Daten irreversibel auf dem nicht-flüchtigen Speicher 52 des bildgebenden medizinischen Gerätes 4 hinterlegt, z.B. mittels einer eFuse und bevorzugt in einem dedizierten, vor einem nachträglichem Eingriff besonders geschützten Speicherbereich. Zusätzlich wird eine digitale Signatur 54 erstellt, welche ebenfalls Daten der zweiten Information 50 beinhaltet. Für jede einzelne Bilddatei 20a wird dabei die digitale Signatur 54 anhand der medizinischen Bildinformation der jeweiligen Bilddatei 20a durch eine entsprechende Hash-Funktion erzeugt, um eine nachträgliche Manipulation auszuschließen. Die digitale Signatur 54 wird in der zugehörigen Bilddatei 20a in den Header eingefügt, und so eine Ausgabedatei 21a erzeugt. Alternativ oder auch zusätzlich hierzu ist es möglich, die medizinische Bildinformation einer Bilddatei 20a mit einem digitalen Wasserzeichen 58 und/oder einem sichtbaren Marker zu versehen, welches bzw. welcher Daten der zweiten Information 50 umfasst. Das Speichern der zweiten Information 50 auch in den jeweiligen Bilddateien 20a ermöglicht auch eine nachträgliche Erfassung der durch die Ausnahme-Nutzungsberechtigung 32 erfolgten Nutzungsvorgangs 22 nur anhand der erzeugten Daten.

Die Bilddaten 20 können nun über die Ausgabedateien 21a auf einem nicht-flüchtigen Speichermedium 56 ausgegeben werden, also z.B. auf einem USB-Stick, einer CD/DVD-ROM, aber auch als ein Papierausdruck der reinen Bildinformation, weswegen die zusätzliche Sicherung der Bilddaten 20 mit einem digitalen Wasserzeichen 58 besonders vorteilhaft ist.

Anhand der zweiten Information 50 kann nachträglich die Nutzung des bildgebenden medizinischen Gerätes 4 durch die Ausnahme-Nutzungsberechtigung 42 insbesondere zum Zweck einer Verrechnung mit der Hersteller-Ebene 16 erfasst werden. Die hier dargestellten Maßnahmen können durch eine Verfahrensroutine 60 kontrolliert werden, welche im nicht-flüchtigen Speicher 52 des bildgebenden medizinischen Gerätes 4 hinterlegt sein kann.

Obwohl die Erfindung im Detail durch das bevorzugte Ausführungsbeispiel näher illustriert und beschrieben wurde, ist die Erfindung nicht durch dieses Ausführungsbeispiel eingeschränkt.

## Patentansprüche

1. Verfahren zur Kontrolle der Nutzung einer medizinischen Infrastruktur (1),
wobei auf einer Hersteller-Ebene (16) der Infrastruktur (1) eine reguläre Nutzungsberechtigung (18) eingerichtet wird, und in einem Normalmodus (30) eine Nutzung der Infrastruktur (1) durch die reguläre Nutzungsberechtigung (18) ermöglicht wird, wobei die reguläre Nutzungsberechtigung (18) einen konkret vorgegebenen Umfang an Funktionen der Infrastruktur (1) zur Nutzung freischaltet,
wobei in einem Notfall (35) auf einer Nutzer-Ebene (8) der Infrastruktur (1) ein Ausnahme-Modus (32) mit einer entsprechenden Ausnahme-Nutzungsberechtigung (42) aktiviert wird, wobei im Ausnahme-Modus (32) eine Nutzung der Infrastruktur (1) auch durch die Ausnahme-Nutzungsberechtigung (42) ermöglicht wird, wobei durch die Ausnahme-Nutzungsberechtigung eine Nutzung sämtlicher von der Konstruktion bzw. Konzeption her möglichen Funktionen der Infrastruktur (1) in beliebigem Umfang und in beliebiger Anzahl ermöglicht wird, wobei der konkret vorgegebene Umfang gegenüber dem beliebigen Umfang eingeschränkt ist, und
wobei im Fall einer Aktivierung des Ausnahme-Modus (32)
- eine den Ausnahme-Modus (32) betreffende erste Information (44) an die Hersteller-Ebene (16) übermittelt wird, und
- eine den Ausnahme-Modus (32) betreffende zweite Information (50) als Dokumentation in einem mit der Infrastruktur (1) verbundenen, ersten nicht-flüchtigen Speichermedium (52, 56) irreversibel gespeichert wird,
wobei bei der Nutzung der Infrastruktur (1) eine Mehrzahl an medizinischen Nutz-Informationen erzeugt wird,
wobei die medizinischen Nutz-Informationen in einer Anzahl an Ausgabedateien (21a) gespeichert werden, und
wobei im Ausnahme-Modus (32) die zweite Information (50) in einer Ausgabedatei (21a) gespeichert wird.

2. Verfahren nach Anspruch 1,
wobei als erste Information (44) und/oder als zweite Information (50) die Aktivierung des Ausnahme-Modus (32) betreffende Daten übermittelt bzw. gespeichert werden.

3. Verfahren nach Anspruch 1 oder Anspruch 2,
wobei ein durch die Ausnahme-Nutzungsberechtigung (42) erfolgter Nutzungsvorgang (22) der Infrastruktur (1) erfasst wird, und
wobei als erste Information (44) und/oder als zweite Information (50) Daten über den derart erfassten Nutzungsvorgang (22) im Ausnahme-Modus (32) übermittelt bzw. gespeichert werden.

4. Verfahren nach einem der vorhergehenden Ansprüche,
wobei ein im Normalmodus (30) durch die reguläre Nutzungsberechtigung (18) erfolgender Nutzungsvorgang (22) erfasst wird, und eine dritte Information (24) über den derart erfassten Nutzungsvorgang (22) im Normalmodus (30) an die Hersteller-Ebene (16) übermittelt wird.

5. Verfahren nach einem der vorhergehenden Ansprüche,
wobei die reguläre Nutzungsberechtigung (18) für eine Nutzung der Infrastruktur (1) im Normalmodus (30) durch einen ersten Lizensierungsmechanismus (26) implementiert wird.

6. Verfahren nach einem der vorhergehenden Ansprüche,
wobei die erste Information (44) über ein Service-Netzwerk (48) übermittelt wird, welches Service-Netzwerk (48) zur Hersteller-seitigen Instandhaltung und/oder Funktionskontrolle der Infrastruktur (1) vorgesehen und eingerichtet ist.

7. Verfahren nach einem der vorhergehenden Ansprüche,
wobei zur Aktivierung des Ausnahme-Modus (32) ein Aktivierungs-Datenschlüssel (38) erzeugt und auf einem zweiten nicht-flüchtigen Speichermedium (40) hinterlegt wird,
wobei die Aktivierung des Ausnahme-Modus (32) anhand des zweiten nicht-flüchtigen Speichermediums (40) erfolgt.

8. Verfahren nach einem der vorhergehenden Ansprüche, wobei
- das erste nicht-flüchtige Speichermedium (52) in der Infrastruktur (1) eingerichtet wird, und/oder
- das erste nicht-flüchtige Speichermedium (56) für eine physikalische Verbindung mit der Infrastruktur (1) vorgesehen und eingerichtet ist,
und die die zweite Information (50) mittels wenigstens eines nicht rücksetzbaren Variablenwertes im ersten nicht-flüchtigen Speichermedium (52, 56) gespeichert wird.

9. Verfahren nach einem der vorhergehenden Ansprüche,
wobei im Ausnahme-Modus (32) die betreffende Ausgabedatei (21a) mit einer digitalen Signatur (54) versehen wird,
wobei die digitale Signatur (54) anhand der jeweiligen medizinischen Nutz-Informationen der Ausgabedatei (21a) erzeugt wird, welche bei der durch die Ausnahme-Nutzungsberechtigung (42) erfolgenden Nutzung der Infrastruktur (1) erzeugt und/oder verarbeitet wurden.

10. Verfahren nach einem der vorhergehenden Ansprüche,
wobei als medizinische Infrastruktur (1) ein bildgebendes medizinisches Gerät (4) genutzt wird, welches dazu eingerichtet ist, von einer Körperregion eines Patienten mittels einer medizinischen Abbildungsmodalität Bilddaten (20) zu erzeugen,
oder
wobei als medizinische Infrastruktur (1) eine medizinische Bilddarstellungs-Software genutzt wird, mittels derer von einem bildgebenden medizinischen Gerät (4) erzeugte Bilddaten (20) auf einer entsprechend eingerichteten Rechnereinheit darstellbar und/oder verarbeitbar sind.

11. Verfahren nach Anspruch 10,
wobei die zweite Information (50) als eine Bildinformation (58) in den erzeugten bzw. verarbeiteten Bilddaten (20) gespeichert wird.

12. Medizinische Vorrichtung (2), welche bei ihrer Nutzung eine Mehrzahl an medizinischen Nutz-Informationen erzeugt, umfassend
- wenigstens eine erste Daten-Schnittstelle und
- einen ersten nicht-flüchtigen Speicher (52),
wobei die medizinische Vorrichtung (2) dazu eingerichtet ist, auf einer Hersteller-Ebene (16) der Infrastruktur (1) eine reguläre Nutzungsberechtigung (18) einzurichten, und
in einem Normalmodus (30) durch die reguläre Nutzungsberechtigung (18) genutzt zu werden, in dem ein konkret vorgegebener Umfang an Funktionen der medizinischen Vorrichtung (2) zur Nutzung freigeschaltet ist,
wobei die medizinische Vorrichtung (2) in einem Notfall (35) auf einer Nutzer-Ebene (8) der Infrastruktur (1) zur Aktivierung eines Ausnahme-Modus (32) mit einer entsprechenden Ausnahme-Nutzungsberechtigung (42) eingerichtet ist, wobei durch die Ausnahme-Nutzungsberechtigung eine Nutzung sämtlicher von der Konstruktion bzw. Konzeption her möglichen Funktionen der medizinischen Vorrichtung (2) in beliebigem Umfang und in beliebiger Anzahl ermöglicht wird, wobei der konkret vorgegebene Umfang gegenüber dem beliebigen Umfang eingeschränkt ist, und
wobei die medizinische Vorrichtung (2) dazu eingerichtet ist, bei aktiviertem Ausnahme-Modus (32)
- eine den Ausnahme-Modus (32) betreffende erste Information (44) über die erste Daten-Schnittstelle auszugeben und an die Hersteller-Ebene (16) zu übermitteln, und
- eine den Ausnahme-Modus (32) betreffende zweite Information (50) als Dokumentation im ersten nicht-flüchtigen Speicher (52) irreversibel zu speichern bzw. in einem an einer zweiten Datenschnittstelle der medizinischen Vorrichtung (2) mit dieser verbundenen, physischen Speichermedium (56) irreversibel zu speichern,
wobei die medizinischen Nutz-Informationen in einer Anzahl an Ausgabedateien (21a) gespeichert werden, und
wobei im Ausnahme-Modus (32) die zweite Information (50) in einer Ausgabedatei (21a) gespeichert wird.

13. Medizinische Vorrichtung (2) nach Anspruch 12, ausgebildet als bildgebendes medizinisches Gerät (4), welche bei der Nutzung als medizinische Nutz-Informationen Bilddaten (20) einer menschlichen Körperregion erzeugt,
wobei das bildgebende medizinische Gerät (4) dazu eingerichtet ist, erzeugte Bilddaten (20) über eine zweite Daten-schnittstelle auszugeben und/oder auf einem nicht-flüchtigen Speichermedium (56) zu speichern,
wobei das bildgebende medizinische Gerät (4) ferner dazu eingerichtet ist, die zweite Information (50) als Bildinformation (58) in den Bilddaten (20) zu speichern.

14. Computerprogrammprodukt mit Programmcode zur Durchführung einer Verfahrensroutine (60), wenn das Computerprogrammprodukt auf einem Rechner ausgeführt wird,
wobei die Verfahrensroutine (60) eine Nutzung einer medizinischen Infrastruktur (1) nach einem Verfahren gemäß einem der der Ansprüche 1 bis 11 kontrolliert.

## Claims

1. Method for monitoring the use of a medical infrastructure (1),
wherein a regular use authorisation (18) is set up at a manufacturer level (16) of the infrastructure (1), and in a normal mode (30) use of the infrastructure (1) is enabled by the regular use authorisation (18), wherein the regular use authorisation (18) unlocks a specifically predefined extent of functions of the infrastructure (1) for use,
wherein, in the event of an emergency (35), an exceptional mode (32) with a corresponding exceptional use authorisation (42) is activated at a user level (8) of the infrastructure (1), wherein, in the exceptional mode (32), use of the infrastructure (1) is also enabled by the exceptional use authorisation (42), wherein the exceptional use authorisation (42) enables use of all the functions of the infrastructure (1) that are possible from the construction or conception, to any desired extent and in any desired number, wherein the specifically predefined extent is restricted compared to the desired extent, and
wherein, in the event of an activation of the exceptional mode (32),
- a first item of information (44) relating to the exceptional mode (32) is transmitted to the manufacturer level (16), and
- a second item of information (50) relating to the exceptional mode (32) is stored on an irreversible basis as documentation in a first non-volatile storage medium (52, 56) that is connected to the infrastructure (1),
wherein a plural number of items of medical use information are generated during use of the infrastructure (1),
wherein the medical use information is stored in a number of output files (21a), and
wherein the second information (50) is stored in an output file (21a) in the exceptional mode (32).

2. Method according to claim 1,
wherein data relating to the activation of the exceptional mode (32) is transmitted or stored as first information (44) and/or as second information (50).

3. Method according to claim 1 or claim 2,
wherein a use procedure (22) of the infrastructure (1) that has taken place by way of the exceptional use authorisation (42) is recorded, and
wherein data regarding the use procedure (22) that has been recorded in such a manner is transmitted or stored in the exceptional mode (32) as first information (44) and/or as second information (50).

4. Method according to one of the preceding claims,
wherein a use procedure (22) that takes place in the normal mode (30) by way of the regular use authorisation (18) is recorded, and a third item of information (24) regarding the use procedure (22) recorded in this manner is transmitted in the normal mode (30) to the manufacturer level (16).

5. Method according to one of the preceding claims,
wherein the regular use authorisation (18) for use of the infrastructure (1) in the normal mode (30) is implemented by a first licensing mechanism (26).

6. Method according to one of the preceding claims,
wherein the first information (44) is transmitted via a service network (48), which service network (48) is provided and set up for the manufacturer-side maintenance and/or function monitoring of the infrastructure (1).

7. Method according to one of the preceding claims,
wherein, for activating the exceptional mode (32), an activation data key (38) is generated and stored on a second non-volatile storage medium (40), wherein the activation of the exceptional mode (32) takes place on the basis of the second non-volatile storage medium (40).

8. Method according to one of the preceding claims, wherein
- the first non-volatile storage medium (52) is set up in the infrastructure (1), and/or
- the first non-volatile storage medium (56) is provided and set up for a physical connection to the infrastructure (1),
and the second information (50) is stored in the first non-volatile storage medium (52, 56) by means of at least one non-resettable variable value.

9. Method according to one of the preceding claims,
wherein, in the exceptional mode (32), the relevant output file (21a) is provided with a digital signature (54), wherein the digital signature (54) is generated on the basis of the respective medical use information of the output file (21a), which has been generated and/or processed during the use of the infrastructure (1) that takes place by way of the exceptional use authorisation (42).

10. Method according to one of the preceding claims,
wherein a medical imaging device (4) is used as medical infrastructure (1) and is set up to generate image data (20) of a body region of a patient by means of a medical mapping modality, or
wherein a piece of medical image representation software is used as medical infrastructure (1), by means of which image data (20) generated by a medical imaging device (4) can be presented and/or processed on a computer unit that has been set up accordingly.

11. Method according to claim 10,
wherein the second information (50) is stored as image information (58) in the generated or processed image data (20).

12. Medical apparatus (2) which, when used, generates a plural number of items of medical use information, comprising
- at least one first data interface and
- a first non-volatile storage device (52),
wherein the medical apparatus (2) is set up to set up a regular use authorisation (18) at a manufacturer level (16) of the infrastructure (1), and to be used in a normal mode (30) by the regular use authorisation (18), in which a specifically predefined extent of functions of the medical apparatus (2) are unlocked for use,
wherein, in the event of an emergency (35), at a user level (8) of the infrastructure (1), the medical apparatus (2) is set up to activate an exceptional mode (32) with a corresponding exceptional use authorisation (42), wherein the exceptional use authorisation enables use of all the functions of the medical apparatus (2) that are possible from the construction or conception, to any desired extent and in any desired number, wherein the specifically predefined extent is restricted compared to the desired extent, and
wherein, when the exceptional mode (32) is activated, the medical apparatus (2) is set up
- to output a first item of information (44) relating to the exceptional mode (32) via the first data interface and to transmit it to the manufacturer level (16), and
- to store a second item of information (50) relating to the exceptional mode (32) on an irreversible basis as documentation in the first non-volatile storage medium (52) or to store it on an irreversible basis in a physical storage medium (56) that is connected thereto on a second data interface of the medical apparatus (2),
wherein the medical use information is stored in a number of output files (21a), and
wherein the second information (50) is stored in an output file (21a) in the exceptional mode (32).

13. Medical apparatus (2) according to claim 12, embodied as a medical imaging device (4), which generates image data (20) of a human body region when used as medical use information,
wherein the medical imaging device (4) is set up to output generated image data (20) via a second data interface and/or to store it on a non-volatile storage medium (56),
wherein the medical imaging device (4) is further set up to store the second information (50) as image information (58) in the image data (20).

14. Computer program product with program code for performing a method routine (60), when the computer program product is executed on a computer,
wherein the method routine (60) monitors a use of a medical infrastructure (1) according to a method according to one of claims 1 to 11.

## Revendications

1. Procédé de contrôle de l'utilisation d'une infrastructure (1) médicale,
dans lequel, sur un plan (16) de fabriquant de l'infrastructure (1), on établit une autorisation (18) régulière d'utilisation, et dans un mode (30) normal, on permet une utilisation de l'infrastructure (1) par l'autorisation (18) régulière d'utilisation, dans lequel l'autorisation (18) régulière d'utilisation libère, à l'utilisation, un périmètre donné à l'avance de manière concrète de fonctions de l'infrastructure (1), dans lequel, dans un cas (35) d'urgence, on active, sur un plan (8) d'utilisateur de l'infrastructure (1), un mode (32) d'exception ayant une autorisation (42) d'utilisation d'exception correspondante,
dans lequel, dans le mode (32) d'exception, on permet une utilisation de l'infrastructure (1) également par l'autorisation (42) d'utilisation d'exception, dans lequel, par l'autorisation d'utilisation d'exception, on permet une utilisation de l'ensemble des fonctions possibles de l'infrastructure (1) par la construction ou la conception en n'importe quel périmètre et en n'importe quel nombre, dans lequel le périmètre, donné à l'avance de manière concrète, est limité par rapport au n'importe quel périmètre, et
dans lequel, dans le cas d'une activation du mode (32) d'exception,
- on transmet, au plan (16) de fabricant, une première information (44) correspondant au mode (32) d'exception, et
- on met en mémoire, de manière irréversible, une deuxième information (50) concernant le mode (32) d'exception, comme documentation dans un premier support (52, 56) de mémoire non volatile relié à l'infrastructure (1),
dans lequel, lors de l'utilisation de l'infrastructure (1), on produit une pluralité d'informations médicales utiles,
dans lequel on met des informations médicales utiles en mémoire dans un certain nombre de fichiers (21a) de sortie, et
dans lequel, dans le mode (32) d'exception, on met la deuxième information (50) en mémoire dans un fichier (21a) de sortie.

2. Procédé suivant la revendication 1,
dans lequel on transmet ou met en mémoire, comme première information (44) et/ou comme deuxième information (50), des données concernant l'activation du mode (32) d'exception.

3. Procédé suivant la revendication 1 ou la revendication 2,
dans lequel on détecte une opération (22) d'utilisation de l'infrastructure (1) effectuée par l'autorisation (42) d'utilisation d'exception, et
dans lequel on transmet ou l'on met en mémoire, dans le mode (32) d'exception, comme première information (44) et/ou comme deuxième information (50), des données sur l'opération (22) d'utilisation ainsi détectée.

4. Procédé suivant l'une des revendications précédentes,
dans lequel on détecte une opération (22) d'utilisation s'effectuant dans le mode (30) normal, par l'autorisation (18) régulière d'utilisation, et on transmet, au plan (16) de fabriquant dans le mode (30) normal, une troisième information (24) sur l'opération (22) d'utilisation ainsi détectée.

5. Procédé suivant l'une des revendications précédentes,
dans lequel on met en œuvre, par un premier mécanisme (26) de licence, l'autorisation (18) régulière d'utilisation pour une utilisation de l'infrastructure (1) dans le mode (30) normal.

6. Procédé suivant l'une des revendications précédentes,
dans lequel on transmet la première information (44) par un réseau (48) de service, lequel réseau (48) de service est prévu et agencé pour l'entretien par le fabricant et/ou le contrôle de fonction de l'infrastructure (1).

7. Procédé suivant l'une des revendications précédentes,
dans lequel, pour l'activation du mode (32) d'exception, on produit une clé (38) de données d'activation et on la met sur un deuxième support (40) de mémoire non volatile,
dans lequel l'activation du mode (32) d'exception s'effectue à l'aide du deuxième support (40) de mémoire non volatile.

8. Procédé suivant l'une des revendications précédentes,
dans lequel
- on met le premier support (52) de mémoire non volatile dans l'infrastructure (1), et/ou
- on prévoit et on agence le premier support (56) de mémoire non volatile pour une liaison physique avec l'infrastructure (1),
et on met la deuxième information (50), au moyen d'au moins une valeur de variable ne pouvant pas être mise à l'état initial, dans le premier support (52, 56) de mémoire non volatile.

9. Procédé suivant l'une des revendications précédentes,
dans lequel, dans le mode (32) d'exception, on munit le fichier (21a) de sortie concerné d'une signature (54) numérique,
dans lequel on produit la signature (54) numérique à l'aide des informations médicales utiles respectives des fichiers (21a) de sortie, qui ont été produits et/ou traités lors de l'utilisation de l'infrastructure (1) s'effectuant par l'autorisation (42) d'utilisation d'exception.

10. Procédé suivant l'une des revendications précédentes,
dans lequel on utilise, comme infrastructure (1) médicale, un appareil (4) médical d'imagerie, qui est agencé pour produire d'une région du corps d'un patient, des données (20) d'image au moyen d'une modalité de reproduction médicale, ou
dans lequel on utilise, comme infrastructure (1) médicale, un logiciel médical de représentation d'image, au moyen duquel on peut représenter et/ou traiter sur une unité informatique agencée en conséquence, des données (20) d'image produites au moyen d'un appareil (4) médical d'imagerie.

11. Procédé suivant la revendication 10,
dans lequel on met en mémoire la deuxième information (50), comme information (58) d'image, dans les données (20) d'image produites ou traitées.

12. Dispositif (2) médical, qui produit, lors de son utilisation, une pluralité d'informations médicales utiles, comprenant
- au moins une première interface de données et
- une première mémoire (52) non volatile,
dans lequel le dispositif (2) médical est agencé pour établir, sur un plan (16) de fabricant de l'infrastructure (1), une autorisation (18) régulière d'utilisation et pour, dans un mode (30) normal, être utilisé par l'autorisation (18) régulière d'utilisation,
dans lequel un périmètre, donné à l'avance de manière concrète, de fonctions du dispositif (2) médical est libéré à l'utilisation, dans lequel le dispositif (2) médical est agencé, dans un cas (35) d'urgence sur un plan (8) d'utilisateur de l'infrastructure (1), pour l'activation d'un mode (32) d'exception par une autorisation (42) d'utilisation d'exception correspondante, dans lequel, par l'autorisation d'utilisation d'exception, on permet une utilisation de l'ensemble des fonctions possibles du dispositif (2) médical par la construction ou la conception en n'importe quel périmètre et en n'importe quel nombre, dans lequel le périmètre, donné à l'avance de manière concrète, est limité par rapport au n'importe quel périmètre, et
dans lequel le dispositif (2) médical est agencé pour, lorsque le mode (32) d'exception est activé,
- émettre et transmettre au plan (16) de fabricant, par l'intermédiaire de la première interface de données, une première information (44) concernant le mode (32) d'exception,
et
- mettre en mémoire, de manière irréversible, comme documentation dans la première mémoire (52) non volatile, une deuxième information (50) concernant le mode (32) d'exception ou la mettre en mémoire, de manière irréversible, dans un support (56) physique de mémoire relié au dispositif (2) médical à une deuxième interface de données de celui-ci,
dans lequel les informations médicales utiles sont mises en mémoire en un certain nombre de fichiers (21a) de sortie, et
dans lequel, dans le mode (32) d'exception, la deuxième information (50) est mise en mémoire dans un fichier (21a) de sortie.

13. Dispositif (2) médical suivant la revendication 12, constitué en appareil (4) médical d'imagerie, qui, lors de l'utilisation, produit, comme informations médicales utiles, des données (20) d'image d'une région du corps humain,
dans lequel l'appareil (4) médical d'imagerie est agencé pour donner des données (20) d'image produites par l'intermédiaire d'une deuxième interface de données et/ou pour les mettre en mémoire sur un support (56) de mémoire non volatile,
dans lequel l'appareil (4) médical d'imagerie est agencé en outre pour mettre en mémoire la deuxième information (50), comme information (58) d'image, dans les données (20) d'image.

14. Produit de programme d'ordinateur ayant un code de programme pour exécuter une routine (60) de procédé, lorsque le produit de programme d'ordinateur est exécuté sur un ordinateur,
dans lequel la routine (60) de procédé commande une utilisation d'une infrastructure (1) médicale suivant un procédé selon l'une des revendications 1 à 11.
